# EUROPEAN PATENT APPLICATION

(11) **EP 3 636 740 A1**
(43) Date of publication of application: **15.04.2020**
(21) Application number: 18813546.1
(22) Date of filing: 04.06.2018
(51) Int. Cl.: C12M 1/02, C12N 1/00, C12M 1/34

(54) **CULTURING APPARATUS AND CULTURING METHOD**

(30) Priority: 05.06.2017 JP 2017110868
(71) Applicant: Dai Nippon Printing Co., Ltd., Tokyo 162-8001 (JP)
(72) Inventor: HAYAKAWA Atsushi, Tokyo 162-8001 (JP); TAMAGAWA Ryuichi, Tokyo 162-8001 (JP)
(74) Representative: Müller-Boré & Partner Patentanwälte PartG mbB
(86) International application number: PCT/JP2018/021429
(87) International publication number: WO 2018/225700

(57) **Abstract**

A culturing apparatus (10) is an apparatus for culturing a culture medium with which an airtight container (35) is filled. Such a culturing apparatus (10) is provided with a container holding unit (12) that holds the airtight container (35) and a motion imparting unit (15) that imparts physical motions from the outside of the airtight container (35) to the airtight container (35) held on the container holding unit (12).

## Description

### Technical Field

The present invention relates to a culturing apparatus and a culturing method.

### Background Art

A sterile filling system (aseptic filling system) has been known in which a sterilized content is filled inside a sterilized container (PET bottle) in a sterile environment and then the container is capped with a cap. Specifically, in the sterile filling system, a molded container is fed to the sterile filling system, and a hydrogen peroxide aqueous solution as a disinfectant is sprayed into the container in the sterile filling system. After that, the container is dried and sterilized, and then, a content is aseptically filled inside the container.

It is necessary to confirm whether or not the sterility of the system is ensured before actual filling of the container is started at an initial stage of such a sterile filling system. For this reason, various tests are being conducted to confirm the sterility of the system. In order to comprehensively evaluate the sterility of the sterile filling system at a final stage after such various tests, an evaluation method using a container containing a culture medium, obtained by filling the container with the culture medium using the sterile filling system, has been performed.

In this case, it is general that the container filled with the culture medium is allowed to stand and cultured in a constant temperature chamber. After a lapse of a predetermined period (for example, 7 days or more), the container filled with the culture medium is taken out of a constant temperature chamber, and whether or not the bacteria survive or propagate in the culture medium in the container is verified. However, conventionally, since the time required for culturing the container in the constant temperature chamber is long, there is a problem in that it is difficult to use the sterile filling system during this period.

### Citation List

### Patent Literature

Patent Literature 1: JP 2010-233563 A

For aerobic microorganisms, oxygen breathing is essential to gain energy. On the other hand, conventionally, as a culturing apparatus for aerobically culturing the microorganisms, there are a culturing apparatus that applies a force to above a culturing container to stir, a culturing apparatus that rotates the culturing container to stir, and the like, and a culturing apparatus that supplies oxygen to microorganisms according to the purpose and conditions has been known.

For example, a culturing apparatus that applies an external force to an upper surface of a culturing container to stir a culture solution has been known (refer to Patent Literature 1). Such a culturing apparatus is considered so as not to prevent the formation and decomposition of agglomerates of microorganisms. However, in the culturing apparatus disclosed in Patent Literature 1, since it is necessary to use a soft material so that the culturing container can be stirred by an external force, there is a restriction in which the stirring cannot be performed in a case of using a culturing container made of a hard material that cannot be deformed by an external force.

In another example, a culturing apparatus that stirs a culture solution by moving a culturing container in the left-right direction (rotating or reciprocating motion) has been known. Such a culturing apparatus is considered to improve the oxygen supply of microorganisms efficiently. However, since the culturing container and a storage container for packing the culturing container needs to be made of a strong material so as to withstand the external force, if the culturing container and the storage container are made of a weak material such as paper, there is a risk that the culturing container and the storage container may be deformed or damaged in some cases by the external force. Therefore, it is substantially difficult to use the above-described culturing apparatus for verification of a sterile filling system, for example.

The present invention has been made in consideration of such points, and an object thereof is to provide a culturing apparatus and a culturing method that can rapidly culture a culture medium in an airtight container by allowing oxygen to be efficiently dissolved in the culture medium in the airtight container.

### Summary of Invention

### Technical Problem

The present invention is a culturing apparatus for culturing a culture medium with which an airtight container is filled, the culturing apparatus includes a container holding unit that holds the airtight container, and a motion imparting unit that imparts, from the outside of the airtight container, a physical motion to the airtight container held in the container holding unit.

According to the present invention, in the culturing apparatus, the container holding unit is provided with a guide portion for preventing or suppressing a fall of the airtight container.

According to the present invention, in the culturing apparatus, the motion imparting unit is a vibration imparting unit that imparts a motion to the airtight container in vertical and horizontal directions, and imparts vibration to the airtight container in a case where the motion is vibration.

According to the present invention, in the culturing apparatus, the number of motions imparted to the airtight container by the motion imparting unit is 600 times/minute or more.

According to the present invention, in the culturing apparatus, a motion width (amplitude in a case where the motion is vibration) of the motion imparted to the airtight container by the motion imparting unit is 5 mm or less.

According to the present invention, in the culturing apparatus, a plurality of the container holding units is provided in a vertical direction, and the airtight containers are held in the respective container holding units.

The present invention is a culturing method for culturing a culture medium with which an airtight container is filled, the method includes a step of holding an airtight container by a container holding unit, and a step of imparting a physical motion, from the outside of the airtight container, to the airtight container held in the container holding unit.

According to the present invention, in the culturing method, the step of imparting the physical motion is a step of imparting a motion (vibration or the like) to the airtight container in vertical and horizontal directions.

According to the present invention, in the culturing method, in the step of imparting the motion (vibration or the like), the number of motions imparted to the airtight container is 600 times/minute or more.

According to the present invention, in the culturing method, in the step of imparting the motion (vibration or the like), a motion width (amplitude in a case of the vibration) of the motion imparted to the airtight container is 5 mm or less.

According to the present invention, the culture medium in the airtight container can be rapidly cultured by making oxygen dissolve efficiently with respect to the culture medium in the airtight container.

### Brief Description of Drawings

Fig. 1 is a schematic cross-sectional view illustrating a culturing apparatus according to one embodiment of the present invention.
Fig. 2 is a schematic plan view illustrating a culturing apparatus according to one embodiment of the present invention.

### Description of Embodiments

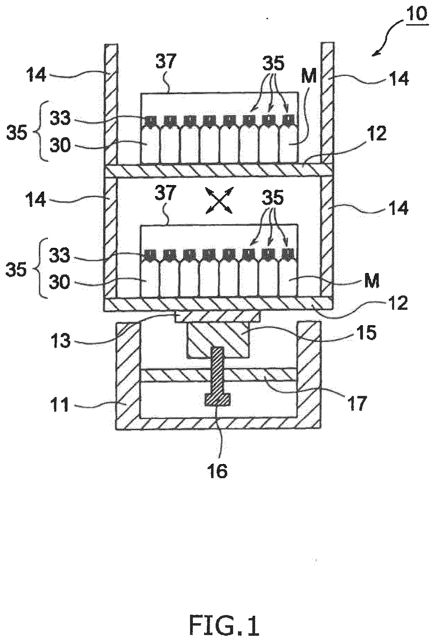

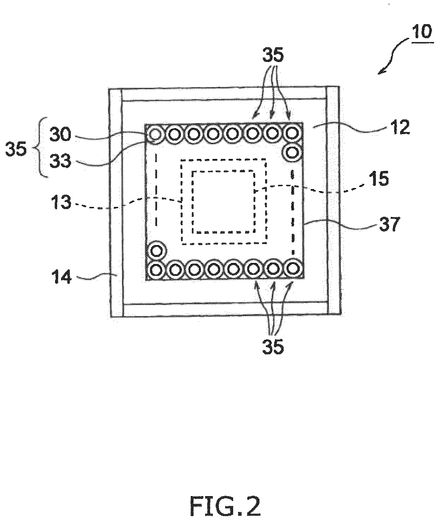

An embodiment of the present invention will be described below with reference to the drawings. Figs. 1 and 2 illustrate one embodiment of the present invention.

### (Culturing apparatus)

First, a culturing apparatus according to the present embodiment will be described with reference to Figs. 1 and 2.

A culturing apparatus 10 as illustrated in Figs. 1 and 2 is an apparatus for culturing a culture medium M with which an airtight container 35 is filled. In particular, the culturing apparatus 10 checks at an early stage whether or not bacteria survive and propagate in the culture medium M in the airtight container 35. Such a culturing apparatus 10 is provided with a table (container holding unit) 12 that holds the airtight container 35 and a vibrator (motion imparting unit, vibration imparting unit) 15 that imparts vibration (motion) from the outside of the airtight container 35 to the airtight container 35 held on the table 12.

In this case, the airtight container 35 includes a bottle (container) 30 and a cap 33 that airtightly seals the bottle 30. The bottle 30 is made by performing biaxial stretching blow molding on a preform made by performing injection molding on a synthetic resin material. A material of the bottle 30 to be used is preferably a thermoplastic resin such as PE (polyethylene), PP (polypropylene), PET (polyethylene-telephthalate), or PEN (polyethylene naphthalate). In addition, the airtight container 35 may be a glass, a can, paper, a pouch, or a composite container of these. The present embodiment will describe an example of a case where a bottle is used for the airtight container 35.

In addition, the airtight container 35 is stored in a storage container 37 such as a box in a state where a plurality of the airtight containers 35 is arranged, and is disposed on the table 12 in this state.

The table 12 is made of a flat planar plate member having a substantially rectangular shape. Further, a plurality (two in this case) of the tables 12 are provided so as to be separated from each other in the vertical direction. Each table 12 holds the airtight container 35 stored in the storage container 37.

The table 12 located below is provided with a guide portion 14 for preventing the airtight container 35 and/or the storage container 37 from falling. Specifically, a plate-like guide portion 14 is erected around the table 12, and each guide portion 14 extends in the vertical direction. Another table 12 is attached above the guide portion 14. The guide portions 14 are provided at both ends of the table 12, respectively. Further, the guide portion 14 is also provided at an end portion in a front side direction and/or a rear side direction in Fig. 1. The table 12 located above is also provided with a guide portion 14 for preventing or suppressing the airtight container 35 and/or the storage container 37 from falling.

For example, the guide portion 14 may be provided on the table 12 so as to be detachable, and the table 12 may be sequentially attached on the guide portion 14. With this, the tables 12 can be arranged in multiple stages, and a large number of airtight containers 35 and/or storage containers 37 can be arranged in the culturing apparatus 10.

The vibrator 15 is attached below the table 12 via a connecting member 13. When the vibrator 15 imparts vibration to the table 12, the table 12 vibrates, and the vibration is also imparted to the airtight container 35 on the table 12. As the vibrator 15, for example, an ultrasonic vibrator or the like may be used. In particular, by using the vibrator 15 as a motion imparting unit, even if the airtight container 35 and the storage container 37 are made of a weak material such as paper, the amplitude is reduced so that the shape is not deformed or damaged by an external force, and oxygen in the airtight container 35 is efficiently dissolved into the culture medium M, and thus the number of motions can be increased.

The number of motions of the vibrations that the vibrator 15 imparts to the table 12 and the airtight container 35 is 600 times/minute or more. By setting the number of motions of the vibrations imparted by the vibrator 15 to be within the above range, oxygen in the airtight container 35 can be efficiently dissolved into the culture medium M, and a culture speed of the culture medium M can be increased.

Further, the amplitude of the vibration that the vibrator 15 imparts to the table 12 and the airtight container 35 is 5 mm or less. By setting the amplitude of the vibration imparted by the vibrator 15 within the above range, the oxygen in the airtight container 35 can be efficiently dissolved into the culture medium M, and even if the airtight container 35 and the storage container 37 are made of a weak material such as paper, the airtight container 35 and the storage container 37 can be prevented from being deformed by an external force, and thereby the culture speed of the culture medium M can be increased.

The vibrator 15 is fixed to a mounting plate 17 using a vibrator fixing bolt 16. The mounting plate 17 is attached to a base 11. The base 11 is placed, for example, on a flat horizontal surface in a thermostatic chamber (not shown).

### (Culturing method)

Next, a culturing method for culturing the culture medium M in the airtight container 35 using the culturing apparatus 10 described above will be described.

The culturing method according to the present embodiment is performed, for example, to check whether or not sterility of the sterile filling system (aseptic filling system) (not shown) is secured. A sterile filling system (aseptic filling system) has been known in which a sterilized content is filled inside a sterilized bottle (PET bottle) 30 in a sterile environment and then the bottle 30 is capped with a cap 33. The culturing method according to the present embodiment is, for example, at an early stage immediately after completion of the above-described sterile filling system, that is, before filling the bottle 30 by actually using the sterile filling system and starting production of the product bottle. Alternatively, the culturing method may be performed in a case where there is a concern of an influence on the sterility such as a case where there occurs a change in a process in the sterile filling system or the device, or in a case where the sterile filling system has not been used for a while. Alternatively, this culturing method may be periodically performed for each predetermined filling cycle, regardless of whether or not there is a risk of affecting sterility.

First, before performing the culturing method according to the present embodiment, a test as to whether or not sterility is ensured for each element of the sterile filling system is individually performed. Specifically, for example, a test as to whether or not a supply line of the content is properly increased in temperature (SIP temperature increase confirmation test), a test as to whether the bottle 30 and the cap 33 are properly sterilized (bottle sterilization test, cap sterilization test), a test as to whether or not the sterile chamber on which the sterile filling system is disposed is sterilized (chamber sterilization test), and the like are performed.

After performing such a test, the sterility of the sterile filling system is verified in order to evaluate the sterility of the bottle 30. Specifically, a large number of the bottles 30 are allowed to flow into the sterile filling system, and each of the bottles 30 is filled with a predetermined culture medium M instead of the content to be actually filled inside each of the bottles 30, and capped by the cap 33. Thereafter, after putting a large number of airtight containers 35 thus prepared in the storage container 37, the entire storage container 37 is placed on the culturing apparatus 10, and the culture medium M in the airtight container 35 is cultured using the culturing apparatus 10. In addition, it is confirmed that the culture medium M in the airtight container 35 does not become corrupted after the lapse of a certain period of time.

Hereinafter, the culturing method according to the present embodiment will be described.

First, in the above-described sterile filling system, a large number of bottles 30 are filled with a sterilized culture medium M and capped by a cap 33 to produce an airtight container 35. The number of the bottles 30 is predetermined, and can be set to a predetermined number, for example 100 or more and 300,000 or less (preferably 1,000 or more and 30,000 or less).

Next, the airtight container 35 filled with the culture medium M is taken out of the sterile filling system and packed in a storage container 37 such as a box. The storage container 37 is tilted (or inverted) manually or automatically on a conveyor, and the culture medium M is securely brought into contact with the inner surface of the airtight container 35.

Next, the airtight container 35 is conveyed to the culturing apparatus 10 while being stored in the storage container 37. For example, the culturing apparatus 10 may be disposed in a constant temperature chamber that is previously maintained at a predetermined temperature of 25°C or higher and 40°C or lower. In addition, when the product bottle to be a target is warmed and sold by a hot bender or the like, it is preferable to confirm sterility against thermophilic bacteria, and the culturing apparatus 10 may be disposed in the constant temperature chamber at a temperature of 40°C or higher and 65°C or lower in addition to the above culture conditions.

Thereafter, the airtight container 35 stored in the storage container 37 is disposed on each table 12 of the culturing apparatus 10 and held on the table 12.

Subsequently, the culturing apparatus 10 is turned on and the vibrator 15 is operated to vibrate the table 12 in the vertical and horizontal directions (arrow directions in Fig. 1). As a result, the vibration is imparted to the airtight container 35 held on the table 12 via the table 12. In this case, the number of vibrations is 600 times/minute or more, and the vibration amplitude (motion width) is 5 mm or less. At this time, the vibrator 15 may always impart vibration to the airtight container 35 with a constant number of motions and/or amplitude, or impart vibration to the airtight container 35 while periodically changing the number of motions and/or amplitude. Alternatively, the vibrator 15 may intermittently impart vibration to the airtight container 35.

After a lapse of a predetermined culture period (for example, three days or more, preferably seven days or more), the culturing apparatus 10 is stopped and all the airtight containers 35 are taken out from the culturing apparatus 10, and whether or not bacteria survive or propagate in the culture medium M in the airtight containers 35 is verified. As a result of this verification, if the number of the airtight containers 35 in which the bacteria survive or propagate is not more than a predetermined number (for example, zero), it is judged that the sterility of the sterile filling system is ensured. On the other hand, as a result of this verification, if the number of the airtight containers 35 in which the bacteria survive or propagate is more than a predetermined number (for example, one or more), it is judged that the sterility of the sterile filling system is insufficient, and measures are taken. For example, the conveyance path and carrying-in path for the bottle 30 may be sterilized, or the sterilization conditions of the bottle 30 in the sterile filling system may be prepared (strengthened).

Thus, by imparting the vibration to the airtight container 35 using the vibrator 15, the physical movement is added to the culture medium M in the airtight container 35, and the airtight container 35 is stored in a state where the culture medium M is moving. With this, the period during which the culture medium M in the airtight container 35 is cultured in the culturing apparatus 10 can be shortened. That is, since dissolution of oxygen into the culture medium M is promoted by adding physical movement to the culture medium M, bacteria are cultured aerobically, whereby culture speed of the bacteria can be increased. As a result, it becomes possible to shorten the predetermined period (for example, three days or more, preferably seven days or more) necessary for culture, and it is possible to promptly judge whether or not the bacteria are generated in the airtight container 35.

For example, according to the experiments of the inventors of the present invention, it has been found that when vibration is applied to the airtight container 35, filled with the culture medium M, using the culturing apparatus 10 (Example A), culture of bacteria can be further promoted as compared with the case where no vibration is applied to the airtight container 35 (Comparative Example B).

Specifically, a plurality of the airtight containers 35 each filled with a liquid neutral culture medium was capped in a state of including falling bacteria floating in a laboratory in the head space of the airtight containers 35, and stored at 22°C to 27°C. Meanwhile, vibration of 3,000 times of motions per minute and amplitude of 1 mm were constantly applied to the airtight container 35 (Example A) of one group by the culturing apparatus 10, and the bottles 30 (Comparative Example B) of another group were allowed to stand still without undergoing vibration. As a result, as shown in the table below, the positive rate increased especially during 2 to 3 days of culture days. This indicates that bacteria can be cultured in a shorter number of days by adding vibration, as compared with the case of applying no vibration to the airtight container 35. Here, the positive rate refers to the ratio of the number of the airtight containers 35 which are positive at a predetermined time to the number (total number of positive bottles) of the airtight containers 35 in which bacteria finally survive or propagate (become positive). Specifically, the positive rate is calculated based on the expression "positive rate (%) = (number of bottles that could be visually confirmed as being positive/total number of positive bottles) × 100". The total number of positive bottles was defined as the number of bottles that could be visually confirmed as being positive after 13 days of culture.

**[Table 1]**

| Positive rate | Day 0 | Day 2 | Day 3 | Day 7 |
|---|---|---|---|---|
| Example A (with vibration) | 0% | 30% | 86% | 100% |
| Example B (without vibration) | 0% | 18% | 33% | 100% |

As described above, according to the present embodiment, the vibrator 15 imparts vibration to the airtight container 35 held on the table 12 from the outside of the airtight container 35. With this, the period during which the culture medium M in the airtight container 35 is cultured in the culturing apparatus 10 can be shortened. As a result, for example, the period during which the sterile filling system that is a target for verifying the sterility cannot be used can be shortened and the sterile filling system can be used efficiently.

Further, according to the present embodiment, since the vibration is imparted from the outside of the airtight container 35 by the vibrator 15, there is no need to deform the airtight container 35 or open the airtight container 35, for example, unlike a culturing apparatus that applies a force to the culturing container to stir the culture medium or a culturing apparatus that directly stirs the culture medium in the culturing container. Furthermore, as long as it can be held on the table 12, the culture medium M can be cultured aerobically without being restricted by the material and packing state of the airtight container 35 and the storage container 37.

Further, according to the present embodiment, the table 12 located above is also provided with a guide portion 14 for preventing or suppressing the airtight container 35 from falling. With this, it is possible to prevent a problem in that the airtight container 35 and the storage container 37 fall from the table 12 while vibration is imparted from the outside of the airtight container 35.

Further, according to the present embodiment, the vibrator 15 imparts the vibration to the airtight container 35, so that the airtight container 35 can be moved finely. As a result, it is possible to more effectively promote the dissolution of oxygen into the culture medium M in the airtight container 35.

Further, according to the present embodiment, the number of motions of vibrations imparted by the vibrator 15 is 600 times/minute or more, and the amplitude of the vibration imparted by the vibrator 15 is 5 mm or less. With this, it is possible to more effectively promote the dissolution of oxygen into the culture medium M in the airtight container 35.

Furthermore, according to the present embodiment, a plurality of tables 12 is provided in the vertical direction, and the airtight container 35 is held on each of the tables 12. Since the vibrations can be imparted collectively to a large number of the airtight containers 35 by arranging the tables 12 in multiple stages in this way, oxygen can be efficiently dissolved into the culture medium M in the large number of airtight containers 35, so that the culture medium M can be efficiently cultured.

In addition to the vibration described above, the motion imparted to the airtight container 35 may be motions of a motion method such as rotation, inversion, and reciprocation used in a conventional culturing apparatus. That is, instead of the vibrator 15 (vibration imparting unit), various motion imparting units (rotational motion imparting unit, inverting motion imparting unit, reciprocating motion imparting unit, and the like) that impart physical motions a rotational motion, an inverting motion, and a reciprocating motion, and the like from the outside of the airtight container 35 may be used. In this case, for example, dissolution of oxygen into the culture medium M is promoted by imparting the physical motion such as rotation, inversion, and reciprocation to the culture medium M, bacteria are cultured aerobically, whereby culture speed of the bacteria can be increased. Table 2 indicates the motion method, the number of motions, and the like in a case of using a motion imparting unit that imparts a physical motion such as a reciprocating motion or a rotational motion in addition to the motion imparting unit including the vibrator 15.

**[Table 2]**

| | Example 1 | Comparative example 1 | Comparative example 2 | Comparative example 3 |
|---|---|---|---|---|
| Motion method | Vibration | Reciprocation | Reciprocation/rotation | Reciprocation |
| Motion direction | Vertical and horizontal directions | Horizontal direction | Horizontal direction | Horizontal direction |
| Number of motions (times/minutes) | 600 or more | 50 or more and 180 or less | 25 or more and 250 or less | 50 or more and 200 or less |
| Motion width | 5 mm or less | 70mm | 10 mm or more and 50 mm or less | 70mm |
| Motion imparting unit | Vibrator | Shaft sliding | Roller slide | Roller slide |

As described above, in the present embodiment, even if the airtight container 35 and the storage container 37 are made of a weak material such as paper, the airtight container 35 and the storage container 37 can be prevented from being deformed by an external force. On the other hand, in the culturing apparatus with a large amplitude as in Comparative Examples 1, 2, and 3, the airtight container 35 and the storage container 37 for packing the airtight container 35 need to be made of a strong material so that it can withstand the external force, and thus in a case where the airtight container 35 and the storage container 37 are made of a weak material such as paper, the airtight container 35 and the storage container 37 may be deformed by the external force, and in some cases (for example, the value of the motion width is changed), it is necessary to adjust the conditions.

In the above description, the case where the culturing apparatus 10 is configured to place the airtight container 35 filled with the culture medium M in the sterile filling system and to culture the culture medium M in the airtight container 35 has been described as an example. However, the use of the culturing apparatus 10 is not limited to this, and can be used for any airtight container 35 filled with the culture medium M. For example, the culturing apparatus 10 may be used for obtaining a product generated in the process of microbial growth.

## Claims

1. A culturing apparatus for culturing a culture medium with which an airtight container is filled, the culturing apparatus comprising:
a container holding unit that holds the airtight container; and
a motion imparting unit that imparts, from the outside of the airtight container, a physical motion to the airtight container held in the container holding unit.

2. The culturing apparatus according to Claim 1, wherein the container holding unit is provided with a guide portion for preventing or suppressing a fall of the airtight container.

3. The culturing apparatus according to Claim 1, wherein the motion imparting unit imparts a motion to the airtight container in vertical and horizontal directions.

4. The culturing apparatus according to Claim 3, wherein the number of motions imparted to the airtight container by the motion imparting unit is 600 times/minute or more.

5. The culturing apparatus according to Claim 3, wherein a motion width of the motion imparted to the airtight container by the motion imparting unit is 5 mm or less.

6. The culturing apparatus according to Claim 1, wherein a plurality of the container holding units is provided in a vertical direction, and the airtight containers are held in the respective container holding units.

7. A culturing method for culturing a culture medium with which an airtight container is filled, the method comprising:
a step of holding an airtight container by a container holding unit; and
a step of imparting a physical motion, from the outside of the airtight container, to the airtight container held in the container holding unit.

8. The culturing method according to Claim 7, wherein the step of imparting the physical motion is a step of imparting a motion to the airtight container in vertical and horizontal directions.

9. The culturing method according to Claim 8, wherein, in the step of imparting the motion, the number of motions imparted to the airtight container is 600 times/minute or more.

10. The culturing method according to Claim 8, wherein, in the step of imparting the motion, a motion width of the motion imparted to the airtight container is 5 mm or less.
